# EUROPEAN PATENT SPECIFICATION

(11) **EP 2 756 076 B1**
(45) Date of publication and mention of the grant of the patent: **26.04.2017**
(21) Application number: 12831162.8
(22) Date of filing: 13.09.2012
(51) Int. Cl.: C12N 9/02, C12N 15/53

(54) **ENZYME VARIANTS WITH IMPROVED PROPERTIES**
ENZYMVARIANTEN MIT VERBESSERTEN EIGENSCHAFTEN
VARIANTES D'ENZYMES À PROPRIÉTÉS AMÉLIORÉES

(30) Priority: 15.09.2011 US 201161535032 P
(43) Date of publication of application: 23.07.2014
(73) Proprietor: MetGen Oy, 20780 Kaarina (FI)
(72) Inventor: BIRIKH, Klara, 20780 Kaarina (FI); AZHAYEV, Alexey, 20780 Kaarina (FI)
(74) Representative: Habets, Winand
(86) International application number: PCT/FI2012/050884
(87) International publication number: WO 2013/038062

(56) References cited:
- EP-A1- 1 826 266
- WO-A1-97/09431
- CN-A- 102 115 722
- DURAO P. ET AL.: 'Proximal mutations at the type 1 copper site of CotA laccase: spectroscopic, redox, kinetic and structural characterization of 1494A and L386A mutants.' THE BIOCHEMICAL JOURNAL vol. 412, no. 2, June 2008, pages 339 - 346, XP055145574
- GUPTA N. ET AL.: 'Laboratory evolution of laccase for substrate specificity.' JOURNAL OF MOLECULAR CATALYSIS B: ENZYMATIC vol. 62, no. 3-4, March 2010, pages 230 - 234, XP026855419

### SEQUENCE LISTING

<110> MetGen Oy
<120> Enzyme variants with improved properties
<130> 2111324Pc
<160> 6
<170> PatentIn version 3.5
<210> 1
   <211> 513
   <212> PRT
   <213> Bacillus subtilis
<400> 1
   Met Thr Leu Glu Lys Phe Val Asp Ala Leu Pro Ile Pro Asp Thr Leu
   1               5                   10                  15
   Lys Pro Val Gln Gln Thr Thr Glu Lys Thr Tyr Tyr Glu Val Thr Met
               20                  25                  30
   Glu Glu Cys Ala His Gln Leu His Arg Asp Leu Pro Pro Thr Arg Leu
           35                  40                  45
   Trp Gly Tyr Asn Gly Leu Phe Pro Gly Pro Thr Ile Glu Val Lys Arg
       50                  55                  60
   Asn Glu Asn Val Tyr Val Lys Trp Met Asn Asn Leu Pro Ser Glu His
   65                  70                  75                  80
   Phe Leu Pro Ile Asp His Thr Ile His His Ser Asp Ser Gln His Glu
                   85                  90                  95
   Glu Pro Glu Val Lys Thr Val Val His Leu His Gly Gly Val Thr Pro
               100                 105                 110
   Asp Asp Ser Asp Gly Tyr Pro Glu Ala Trp Phe Ser Lys Asp Phe Glu
           115                 120                 125
   Gln Thr Gly Pro Tyr Phe Lys Arg Glu Val Tyr His Tyr Pro Asn Gln
       130                 135                 140
   Gln Arg Gly Ala Ile Leu Trp Tyr His Asp His Ala Met Ala Leu Thr
   145                 150                 155                 160
   Arg Leu Asn Val Tyr Ala Gly Leu Val Gly Asp Tyr Ile Ile His Asp
                   165                 170                 175
   Pro Lys Glu Lys Arg Leu Lys Leu Pro Ser Gly Glu Tyr Asp Val Pro
               180                 185                 190
   Leu Leu Ile Thr Asp Arg Thr Ile Asn Glu Asp Gly Ser Leu Phe Tyr
           195                 200                 205
   Pro Ser Gly Pro Glu Asn Pro Ser Pro Ser Leu Pro Lys Pro Ser Ile
       210                 215                 220
   Val Pro Ala Phe Cys Gly Asp Thr Ile Leu Val Asn Gly Lys Val Trp
   225                 230                 235                 240
   Pro Tyr Leu Glu Val Glu Pro Arg Lys Tyr Arg Phe Arg Val Ile Asn
                   245                 250                 255
   Ala Ser Asn Thr Arg Thr Tyr Asn Leu Ser Leu Asp Asn Gly Gly Glu
               260                 265                 270
   Phe Ile Gln Ile Gly Ser Asp Gly Gly Leu Leu Pro Arg Ser Val Lys
           275                 280                 285
   Leu Asn Ser Phe Ser Leu Ala Pro Ala Glu Arg Tyr Asp Ile Ile Ile
       290                 295                 300
   Asp Phe Thr Ala Tyr Glu Gly Glu Ser Ile Ile Leu Ala Asn Ser Glu
   305                 310                 315                 320
   Gly Cys Gly Gly Asp Ala Asn Pro Glu Thr Asp Ala Asn Ile Met Gln
                   325                 330                 335
   Phe Arg Val Thr Lys Pro Leu Ala Gln Lys Asp Glu Ser Arg Lys Pro
               340                 345                 350
   Lys Tyr Leu Ala Ser Tyr Pro Ser Val Gln Asn Glu Arg Ile Gln Asn
           355                 360                 365
   Ile Arg Thr Leu Lys Leu Ala Gly Thr Gln Asp Glu Tyr Gly Arg Pro
       370                 375                 380
   Val Leu Leu Leu Asn Asn Lys Arg Trp His Asp Pro Val Thr Glu Ala
   385                 390                 395                 400
   Pro Lys Ala Gly Thr Thr Glu Ile Trp Ser Ile Val Asn Pro Thr Gln
                   405                 410                 415
   Gly Thr His Pro Ile His Leu His Leu Val Ser Phe Arg Val Leu Asp
               420                 425                 430
   Arg Arg Pro Phe Asp Ile Ala Arg Tyr Gln Glu Arg Gly Glu Leu Ser
           435                 440                 445
   Tyr Thr Gly Pro Ala Val Pro Pro Pro Pro Ser Glu Lys Gly Trp Lys
       450                 455                 460
   Asp Thr Ile Gln Ala His Ala Gly Glu Val Leu Arg Ile Ala Val Thr
   465                 470                 475                 480
   Phe Gly Pro Tyr Ser Gly Arg Tyr Val Trp His Cys His Ile Leu Glu
                   485                 490                 495
   His Glu Asp Tyr Asp Met Met Arg Pro Met Asp Ile Thr Asp Pro His
               500                 505                 510
   Lys
<210> 2
   <211> 539
   <212> PRT
   <213> Bacillus subtilis
<400> 2
   Met Thr Leu Glu Lys Phe Val Asp Ala Leu Pro Ile Pro Asp Thr Leu
   1               5                   10                  15
   Lys Pro Val Gln Gln Ser Lys Glu Lys Thr Tyr Tyr Glu Val Thr Met
               20                  25                  30
   Glu Glu Cys Thr His Gln Leu His Arg Asp Leu Pro Pro Thr Arg Leu
           35                  40                  45
   Trp Gly Tyr Asn Gly Leu Phe Pro Gly Pro Thr Ile Glu Val Lys Arg
       50                  55                  60
   Asn Glu Asn Val Tyr Val Lys Trp Met Asn Asn Leu Pro Ser Thr His
   65                  70                  75                  80
   Phe Leu Pro Ile Asp His Thr Ile His His Ser Asp Ser Gln His Glu
                   85                  90                  95
   Glu Pro Glu Val Lys Thr Val Val His Leu His Gly Gly Val Thr Pro
               100                 105                 110
   Asp Asp Ser Asp Gly Tyr Pro Glu Ala Trp Phe Ser Lys Asp Phe Glu
           115                 120                 125
   Gln Thr Gly Pro Tyr Phe Lys Arg Glu Val Tyr His Tyr Pro Asn Gln
       130                 135                 140
   Gln Arg Gly Ala Ile Leu Trp Tyr His Asp His Ala Met Ala Leu Thr
   145                 150                 155                 160
   Arg Leu Asn Val Tyr Ala Gly Leu Val Gly Ala Tyr Ile Ile His Asp
                   165                 170                 175
   Pro Lys Glu Lys Arg Leu Lys Leu Pro Ser Glu Glu Tyr Asp Val Pro
               180                 185                 190
   Leu Leu Ile Thr Asp Arg Thr Ile Asn Glu Asp Gly Ser Leu Phe Tyr
           195                 200                 205
   Pro Ser Gly Pro Glu Asn Pro Ser Pro Ser Leu Pro Asn Pro Ser Ile
       210                 215                 220
   Val Pro Ala Phe Cys Gly Glu Thr Ile Leu Val Asn Gly Lys Val Trp
   225                 230                 235                 240
   Pro Tyr Leu Glu Val Glu Pro Arg Lys Tyr Arg Phe Arg Val Ile Asn
                   245                 250                 255
   Ala Ser Asn Thr Arg Thr Tyr Asn Leu Ser Leu Asp Asn Gly Gly Glu
               260                 265                 270
   Phe Ile Gln Ile Gly Ser Asp Gly Gly Leu Leu Pro Arg Ser Val Lys
           275                 280                 285
   Leu Thr Ser Phe Ser Leu Ala Pro Ala Glu Arg Tyr Asp Ile Ile Ile
       290                 295                 300
   Asp Phe Thr Ala Tyr Glu Gly Gln Ser Ile Ile Leu Ala Asn Ser Ala
   305                 310                 315                 320
   Gly Cys Gly Gly Asp Val Asn Pro Glu Thr Asp Ala Asn Ile Met Gln
                   325                 330                 335
   Phe Arg Val Thr Lys Pro Leu Ala Gln Lys Asp Glu Ser Arg Lys Pro
               340                 345                 350
   Lys Tyr Leu Ala Ser Tyr Pro Ser Val Gln Asn Glu Arg Ile Gln Asn
           355                 360                 365
   Ile Arg Thr Leu Lys Leu Ala Gly Thr Gln Asp Glu Tyr Gly Arg Pro
       370                 375                 380
   Val Leu Leu Leu Asn Asn Lys Arg Trp His Asp Pro Val Thr Glu Ala
   385                 390                 395                 400
   Pro Lys Ala Gly Thr Thr Glu Ile Trp Ser Ile Ile Asn Pro Thr Arg
                   405                 410                 415
   Gly Thr His Pro Ile His Leu His Leu Val Ser Phe Arg Val Ile Asp
               420                 425                 430
   Arg Arg Pro Phe Asp Ile Ala His Tyr Gln Glu Ser Gly Ala Leu Ser
           435                 440                 445
   Tyr Thr Gly Pro Ala Val Pro Pro Pro Pro Ser Glu Lys Gly Trp Lys
       450                 455                 460
   Asp Thr Ile Gln Ala His Ala Gly Glu Val Leu Arg Ile Ala Ala Thr
   465                 470                 475                 480
   Phe Gly Pro Tyr Ser Gly Arg Tyr Val Trp His Cys His Ile Leu Glu
                   485                 490                 495
   His Glu Asp Tyr Asp Met Met Arg Pro Met Asp Ile Thr Asp Pro His
               500                 505                 510
   Lys Ser Asp Pro Asn Ser Ser Ser Val Asp Lys Leu His Arg Thr Arg
           515                 520                 525
   Ala Pro Pro Pro Pro Pro Leu Arg Ser Gly Cys
       530                 535
<210> 3
   <211> 513
   <212> PRT
   <213> Artificial Sequence
<220>
   <223> COT1 introduced with Q386 and P487/W488/F489
<400> 3
   Met Thr Leu Glu Lys Phe Val Asp Ala Leu Pro Ile Pro Asp Thr Leu
   1               5                   10                  15
   Lys Pro Val Gln Gln Thr Thr Glu Lys Thr Tyr Tyr Glu Val Thr Met
               20                  25                  30
   Glu Glu Cys Ala His Gln Leu His Arg Asp Leu Pro Pro Thr Arg Leu
           35                  40                  45
   Trp Gly Tyr Asn Gly Leu Phe Pro Gly Pro Thr Ile Glu Val Lys Arg
       50                  55                  60
   Asn Glu Asn Val Tyr Val Lys Trp Met Asn Asn Leu Pro Ser Glu His
   65                  70                  75                  80
   Phe Leu Pro Ile Asp His Thr Ile His His Ser Asp Ser Gln His Glu
                   85                  90                  95
   Glu Pro Glu Val Lys Thr Val Val His Leu His Gly Gly Val Thr Pro
               100                 105                 110
   Asp Asp Ser Asp Gly Tyr Pro Glu Ala Trp Phe Ser Lys Asp Phe Glu
           115                 120                 125
   Gln Thr Gly Pro Tyr Phe Lys Arg Glu Val Tyr His Tyr Pro Asn Gln
       130                 135                 140
   Gln Arg Gly Ala Ile Leu Trp Tyr His Asp His Ala Met Ala Leu Thr
   145                 150                 155                 160
   Arg Leu Asn Val Tyr Ala Gly Leu Val Gly Asp Tyr Ile Ile His Asp
                   165                 170                 175
   Pro Lys Glu Lys Arg Leu Lys Leu Pro Ser Gly Glu Tyr Asp Val Pro
               180                 185                 190
   Leu Leu Ile Thr Asp Arg Thr Ile Asn Glu Asp Gly Ser Leu Phe Tyr
           195                 200                 205
   Pro Ser Gly Pro Glu Asn Pro Ser Pro Ser Leu Pro Lys Pro Ser Ile
       210                 215                 220
   Val Pro Ala Phe Cys Gly Asp Thr Ile Leu Val Asn Gly Lys Val Trp
   225                 230                 235                 240
   Pro Tyr Leu Glu Val Glu Pro Arg Lys Tyr Arg Phe Arg Val Ile Asn
                   245                 250                 255
   Ala Ser Asn Thr Arg Thr Tyr Asn Leu Ser Leu Asp Asn Gly Gly Glu
               260                 265                 270
   Phe Ile Gln Ile Gly Ser Asp Gly Gly Leu Leu Pro Arg Ser Val Lys
           275                 280                 285
   Leu Asn Ser Phe Ser Leu Ala Pro Ala Glu Arg Tyr Asp Ile Ile Ile
       290                 295                 300
   Asp Phe Thr Ala Tyr Glu Gly Glu Ser Ile Ile Leu Ala Asn Ser Glu
   305                 310                 315                 320
   Gly Cys Gly Gly Asp Ala Asn Pro Glu Thr Asp Ala Asn Ile Met Gln
                   325                 330                 335
   Phe Arg Val Thr Lys Pro Leu Ala Gln Lys Asp Glu Ser Arg Lys Pro
               340                 345                 350
   Lys Tyr Leu Ala Ser Tyr Pro Ser Val Gln Asn Glu Arg Ile Gln Asn
           355                 360                 365
   Ile Arg Thr Leu Lys Leu Ala Gly Thr Gln Asp Glu Tyr Gly Arg Pro
       370                 375                 380
   Val Gln Leu Leu Asn Asn Lys Arg Trp His Asp Pro Val Thr Glu Ala
   385                 390                 395                 400
   Pro Lys Ala Gly Thr Thr Glu Ile Trp Ser Ile Val Asn Pro Thr Gln
                   405                 410                 415
   Gly Thr His Pro Ile His Leu His Leu Val Ser Phe Arg Val Leu Asp
               420                 425                 430
   Arg Arg Pro Phe Asp Ile Ala Arg Tyr Gln Glu Arg Gly Glu Leu Ser
           435                 440                 445
   Tyr Thr Gly Pro Ala Val Pro Pro Pro Pro Ser Glu Lys Gly Trp Lys
       450                 455                 460
   Asp Thr Ile Gln Ala His Ala Gly Glu Val Leu Arg Ile Ala Val Thr
   465                 470                 475                 480
   Phe Gly Pro Tyr Ser Gly Pro Trp Phe Trp His Cys His Ile Leu Glu
                   485                 490                 495
   His Glu Asp Tyr Asp Met Met Arg Pro Met Asp Ile Thr Asp Pro His
               500                 505                 510
   Lys
<210> 4
   <211> 513
   <212> PRT
   <213> Artificial Sequence
<220>
   <223> COT1 introduced with Q386
<400> 4
   Met Thr Leu Glu Lys Phe Val Asp Ala Leu Pro Ile Pro Asp Thr Leu
   1               5                   10                  15
   Lys Pro Val Gln Gln Thr Thr Glu Lys Thr Tyr Tyr Glu Val Thr Met
               20                  25                  30
   Glu Glu Cys Ala His Gln Leu His Arg Asp Leu Pro Pro Thr Arg Leu
           35                  40                  45
   Trp Gly Tyr Asn Gly Leu Phe Pro Gly Pro Thr Ile Glu Val Lys Arg
       50                  55                  60
   Asn Glu Asn Val Tyr Val Lys Trp Met Asn Asn Leu Pro Ser Glu His
   65                  70                  75                  80
   Phe Leu Pro Ile Asp His Thr Ile His His Ser Asp Ser Gln His Glu
                   85                  90                  95
   Glu Pro Glu Val Lys Thr Val Val His Leu His Gly Gly Val Thr Pro
               100                 105                 110
   Asp Asp Ser Asp Gly Tyr Pro Glu Ala Trp Phe Ser Lys Asp Phe Glu
           115                 120                 125
   Gln Thr Gly Pro Tyr Phe Lys Arg Glu Val Tyr His Tyr Pro Asn Gln
       130                 135                 140
   Gln Arg Gly Ala Ile Leu Trp Tyr His Asp His Ala Met Ala Leu Thr
   145                 150                 155                 160
   Arg Leu Asn Val Tyr Ala Gly Leu Val Gly Asp Tyr Ile Ile His Asp
                   165                 170                 175
   Pro Lys Glu Lys Arg Leu Lys Leu Pro Ser Gly Glu Tyr Asp Val Pro
               180                 185                 190
   Leu Leu Ile Thr Asp Arg Thr Ile Asn Glu Asp Gly Ser Leu Phe Tyr
           195                 200                 205
   Pro Ser Gly Pro Glu Asn Pro Ser Pro Ser Leu Pro Lys Pro Ser Ile
       210                 215                 220
   Val Pro Ala Phe Cys Gly Asp Thr Ile Leu Val Asn Gly Lys Val Trp
   225                 230                 235                 240
   Pro Tyr Leu Glu Val Glu Pro Arg Lys Tyr Arg Phe Arg Val Ile Asn
                   245                 250                 255
   Ala Ser Asn Thr Arg Thr Tyr Asn Leu Ser Leu Asp Asn Gly Gly Glu
               260                 265                 270
   Phe Ile Gln Ile Gly Ser Asp Gly Gly Leu Leu Pro Arg Ser Val Lys
           275                 280                 285
   Leu Asn Ser Phe Ser Leu Ala Pro Ala Glu Arg Tyr Asp Ile Ile Ile
       290                 295                 300
   Asp Phe Thr Ala Tyr Glu Gly Glu Ser Ile Ile Leu Ala Asn Ser Glu
   305                 310                 315                 320
   Gly Cys Gly Gly Asp Ala Asn Pro Glu Thr Asp Ala Asn Ile Met Gln
                   325                 330                 335
   Phe Arg Val Thr Lys Pro Leu Ala Gln Lys Asp Glu Ser Arg Lys Pro
               340                 345                 350
   Lys Tyr Leu Ala Ser Tyr Pro Ser Val Gln Asn Glu Arg Ile Gln Asn
           355                 360                 365
   Ile Arg Thr Leu Lys Leu Ala Gly Thr Gln Asp Glu Tyr Gly Arg Pro
       370                 375                 380
   Val Gln Leu Leu Asn Asn Lys Arg Trp His Asp Pro Val Thr Glu Ala
   385                 390                 395                 400
   Pro Lys Ala Gly Thr Thr Glu Ile Trp Ser Ile Val Asn Pro Thr Gln
                   405                 410                 415
   Gly Thr His Pro Ile His Leu His Leu Val Ser Phe Arg Val Leu Asp
               420                 425                 430
   Arg Arg Pro Phe Asp Ile Ala Arg Tyr Gln Glu Arg Gly Glu Leu Ser
           435                 440                 445
   Tyr Thr Gly Pro Ala Val Pro Pro Pro Pro Ser Glu Lys Gly Trp Lys
       450                 455                 460
   Asp Thr Ile Gln Ala His Ala Gly Glu Val Leu Arg Ile Ala Val Thr
   465                 470                 475                 480
   Phe Gly Pro Tyr Ser Gly Arg Tyr Val Trp His Cys His Ile Leu Glu
   485 490 495
   His Glu Asp Tyr Asp Met Met Arg Pro Met Asp Ile Thr Asp Pro His
   500 505 510
   Lys
<210> 5
   <211> 513
   <212> PRT
   <213> Artificial Sequence
<220>
   <223> COT1 introduced with P487/W488/F489
<400> 5
   Met Thr Leu Glu Lys Phe Val Asp Ala Leu Pro Ile Pro Asp Thr Leu
   1               5                   10                  15
   Lys Pro Val Gln Gln Thr Thr Glu Lys Thr Tyr Tyr Glu Val Thr Met
               20                  25                  30
   Glu Glu Cys Ala His Gln Leu His Arg Asp Leu Pro Pro Thr Arg Leu
           35                  40                  45
   Trp Gly Tyr Asn Gly Leu Phe Pro Gly Pro Thr Ile Glu Val Lys Arg
       50                  55                  60
   Asn Glu Asn Val Tyr Val Lys Trp Met Asn Asn Leu Pro Ser Glu His
   65                  70                  75                  80
   Phe Leu Pro Ile Asp His Thr Ile His His Ser Asp Ser Gln His Glu
                   85                  90                  95
   Glu Pro Glu Val Lys Thr Val Val His Leu His Gly Gly Val Thr Pro
               100                 105                 110
   Asp Asp Ser Asp Gly Tyr Pro Glu Ala Trp Phe Ser Lys Asp Phe Glu
           115                 120                 125
   Gln Thr Gly Pro Tyr Phe Lys Arg Glu Val Tyr His Tyr Pro Asn Gln
       130                 135                 140
   Gln Arg Gly Ala Ile Leu Trp Tyr His Asp His Ala Met Ala Leu Thr
   145                 150                 155                 160
   Arg Leu Asn Val Tyr Ala Gly Leu Val Gly Asp Tyr Ile Ile His Asp
                   165                 170                 175
   Pro Lys Glu Lys Arg Leu Lys Leu Pro Ser Gly Glu Tyr Asp Val Pro
   180 185 190
   Leu Leu Ile Thr Asp Arg Thr Ile Asn Glu Asp Gly Ser Leu Phe Tyr
   195 200 205
   Pro Ser Gly Pro Glu Asn Pro Ser Pro Ser Leu Pro Lys Pro Ser Ile
   210 215 220
   Val Pro Ala Phe Cys Gly Asp Thr Ile Leu Val Asn Gly Lys Val Trp
   225 230 235 240
   Pro Tyr Leu Glu Val Glu Pro Arg Lys Tyr Arg Phe Arg Val Ile Asn
   245 250 255
   Ala Ser Asn Thr Arg Thr Tyr Asn Leu Ser Leu Asp Asn Gly Gly Glu
   260 265 270
   Phe Ile Gln Ile Gly Ser Asp Gly Gly Leu Leu Pro Arg Ser Val Lys
   275 280 285
   Leu Asn Ser Phe Ser Leu Ala Pro Ala Glu Arg Tyr Asp Ile Ile Ile
   290 295 300
   Asp Phe Thr Ala Tyr Glu Gly Glu Ser Ile Ile Leu Ala Asn Ser Glu
   305 310 315 320
   Gly Cys Gly Gly Asp Ala Asn Pro Glu Thr Asp Ala Asn Ile Met Gln
   325 330 335
   Phe Arg Val Thr Lys Pro Leu Ala Gln Lys Asp Glu Ser Arg Lys Pro
   340 345 350
   Lys Tyr Leu Ala Ser Tyr Pro Ser Val Gln Asn Glu Arg Ile Gln Asn
   355 360 365
   Ile Arg Thr Leu Lys Leu Ala Gly Thr Gln Asp Glu Tyr Gly Arg Pro
   370 375 380
   Val Leu Leu Leu Asn Asn Lys Arg Trp His Asp Pro Val Thr Glu Ala
   385 390 395 400
   Pro Lys Ala Gly Thr Thr Glu Ile Trp Ser Ile Val Asn Pro Thr Gln
   405 410 415
   Gly Thr His Pro Ile His Leu His Leu Val Ser Phe Arg Val Leu Asp
                   420                 425                 430
   Arg Arg Pro Phe Asp Ile Ala Arg Tyr Gln Glu Arg Gly Glu Leu Ser
           435                 440                 445
   Tyr Thr Gly Pro Ala Val Pro Pro Pro Pro Ser Glu Lys Gly Trp Lys
       450                 455                 460
   Asp Thr Ile Gln Ala His Ala Gly Glu Val Leu Arg Ile Ala Val Thr
   465                 470                 475                 480
   Phe Gly Pro Tyr Ser Gly Pro Trp Phe Trp His Cys His Ile Leu Glu
                   485                 490                 495
   His Glu Asp Tyr Asp Met Met Arg Pro Met Asp Ile Thr Asp Pro His
               500                 505                 510
   Lys
<210> 6
   <211> 451
   <212> PRT
   <213> Artificial Sequence
<220>
   <223> Consensus sequence
<400> 6
   Met Thr Leu Glu Lys Phe Val Asp Ala Leu Pro Ile Pro Asp Thr Leu
   1               5                   10                  15
   Lys Pro Val Ser Lys Thr Tyr Tyr Glu Val Thr Met Glu His Lys Leu
               20                  25                  30
   His Arg Asp Leu Pro Pro Thr Arg Leu Trp Gly Tyr Asn Gly Leu Phe
           35                  40                  45
   Pro Gly Pro Thr Ile Glu Val Arg Asn Glu Val Tyr Val Lys Trp Met
       50                  55                  60
   Asn Asn Leu Pro His Phe Leu Pro Val Asp His Thr Ile His Ser Asp
   65                  70                  75                  80
   His Asp Glu Pro Glu Val Lys Thr Val Val His Leu His Gly Gly Thr
                   85                  90                  95
   Pro Asp Ser Asp Gly Tyr Pro Glu Ala Trp Phe Thr Lys Asp Phe Gln
               100                 105                 110
   Thr Gly Pro Tyr Phe Arg Glu Val Tyr His Tyr Pro Asn Gln Arg Gly
           115                 120                 125
   Ala Leu Trp Tyr His Asp His Ala Met Ala Leu Thr Arg Leu Asn Val
       130                 135                 140
   Tyr Ala Gly Leu Ala Gly Tyr Ile Ile Arg Asp Lys Glu Lys Arg Leu
   145                 150                 155                 160
   Leu Pro Ser Gly Glu Tyr Asp Ile Pro Leu Leu Ile Asp Arg Thr Phe
                   165                 170                 175
   Asn Glu Asp Gly Ser Leu Phe Tyr Pro Ser Pro Glu Asn Pro Ser Pro
               180                 185                 190
   Ser Leu Pro Pro Ser Ile Val Pro Ala Phe Cys Gly Glu Thr Ile Leu
           195                 200                 205
   Val Asn Gly Lys Val Trp Pro Tyr Leu Glu Val Glu Pro Arg Lys Tyr
       210                 215                 220
   Arg Phe Arg Ile Leu Asn Ala Ser Asn Thr Arg Thr Tyr Leu Ser Leu
   225                 230                 235                 240
   Asp Asn Gly Gly Phe Ile Gln Ile Gly Ser Asp Gly Gly Leu Leu Arg
                   245                 250                 255
   Pro Val Lys Leu Asn Ser Leu Ser Leu Ala Pro Ala Glu Arg Asp Ile
               260                 265                 270
   Ile Ile Asp Phe Ser Ala Tyr Glu Gly Ser Ile Ile Leu Asn Gly Cys
           275                 280                 285
   Gly Asp Pro Glu Thr Asp Ala Asn Ile Met Gln Phe Arg Val Thr Lys
       290                 295                 300
   Pro Leu Ala Lys Asp Ser Arg Ile Pro Lys Leu Ala Ile Pro Leu Arg
   305                 310                 315                 320
   Ile Ile Arg Leu Lys Leu Gly Thr Asp Glu Tyr Gly Arg Pro Val Leu
                   325                 330                 335
   Leu Leu Asn Asn Lys Arg Trp His Asp Pro Val Thr Glu Pro Lys Leu
               340                 345                 350
   Gly Thr Thr Glu Ile Trp Ser Ile Ile Asn Thr Gly Thr His Pro Ile
                   355                 360                 365
   His Leu His Leu Val Gln Phe Arg Val Leu Asp Arg Arg Pro Phe Asp
       370                 375                 380
   Ile Arg Tyr Gln Glu Gly Glu Leu Tyr Thr Gly Pro Ala Val Pro Pro
   385                 390                 395                 400
   Pro Ser Glu Lys Gly Trp Lys Asp Thr Val Gln Ala Ala Gly Glu Val
                   405                 410                 415
   Leu Arg Ile Ile Ala Phe Gly Pro Tyr Ser Gly Arg Tyr Val Trp His
               420                 425                 430
   Cys His Ile Leu Glu His Glu Asp Tyr Asp Met Met Arg Pro Met Asp
           435                 440                 445
   Ile Ile Asp
       450

## Description

### FIELD OF THE INVENTION

The present invention relates to laccase variants and uses thereof as eco-friendly biocatalysts in various industrial processes.

### BACKGROUND OF THE INVENTION

Laccases (EC 1.10.3.2) are enzymes having a wide taxonomic distribution and belonging to the group of multicopper oxidases. Laccases are eco-friendly catalysts, which use molecular oxygen from air to oxidize various phenolic and non-phenolic lignin-related compounds as well as highly recalcitrant environmental pollutants, and produce water as the only side-product. These natural "green" catalysts are used for diverse industrial applications including the detoxification of industrial effluents, mostly from the paper and pulp, textile and petrochemical industries, use as bioremediation agent to clean up herbicides, pesticides and certain explosives in soil. Laccases are also used as cleaning agents for certain water purification systems. In addition, their capacity to remove xenobiotic substances and produce polymeric products makes them a useful tool for bioremediation purposes. Another large proposed application area of laccases is biomass pretreatment in biofuel and pulp and paper industry.

Laccases have a wide substrate specificity and they can oxidize many different substrate compounds. Owing to chemical properties of the substrates, they become more readily oxidized in different pH conditions, either alkaline or acidic. On the other hand, the advantageous pH range of action of different laccases may vary, which means that they have a preference to substrates within that range. For instance, relatives of CotA laccase are known to work best in acidic conditions.

A wider operable pH range would be an important feature in laccases, especially in waste water and remediation applications, as acidity of these environments may vary significantly. This feature is also critical for biomass pre-treatment processes, which in certain cases are carried out under alkaline conditions. Thus, there is an identified need in the art for developing laccase variants having a wider pH range of action.

### BRIEF DESCRIPTION OF THE INVENTION

In one aspect, the present disclosure relates to laccase variants, which comprise a glutamine residue situated within 6 Angstrom (A) distance to the type 1 Copper ion in the 3-dimentional structure of the laccase variant.
As described herein, the laccase variant may comprise an amino acid sequence showing at least 50% identity to an amino acid sequence selected from the group consisting of SEQ ID NO:3, SEQ ID NO:4, and SEQ ID NO:5, comprising at least one amino acid variant selected from the group consisting of glutamine (Q) in a position which corresponds to the position 386 of the amino acid sequence depicted in SEQ ID NO:3 and a Proline-Tryptophan-Phenylalanine (PWF) sequence in a position which corresponds to the position 487-489 of the amino acid sequence depicted in SEQ ID NO:3.

As shown herein, the present laccase laccase variants have an increased enzymatic activity in alkaline conditions as compared to that of a corresponding control enzyme lacking said amino acid variants.

The present invention also relates to nucleic acid molecules encoding the present laccase variants, vectors comprising said nucleic acid molecules, and recombinant host cells comprising said vector.

As also described herein, the invention relates to a method of producing the present laccase variants. The method comprises the steps of i) culturing a recombinant host cell according to the present invention under conditions suitable for the production of the laccase variant, and ii) recovering the laccase variant obtained.

In further aspects, the invention relates to various uses of the present laccase variants, especially in pulp delignification, textile dye bleaching, wastewater detoxifixation, and xenobiotic detoxification.

Other specific embodiments, objects, details, and advantages of the invention are set forth in the dependent claims, following drawings, detailed description and examples.

### BRIEF DESCRIPTION OF THE DRAWINGS

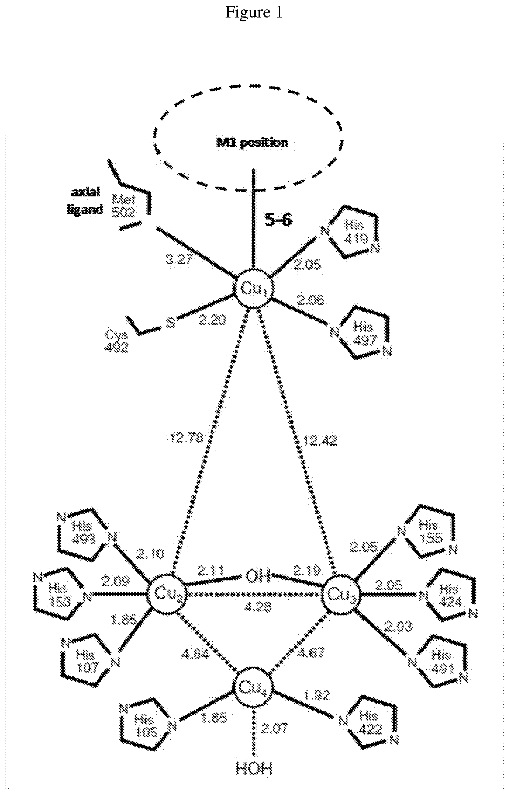

In the following the invention will be described in greater detail by means of preferred embodiments with reference to the attached drawings, in which
Figure 1 is a schematic representation of T1 (Cu1) and T2/T3 (Cu4/Cu2-Cu3) copper sites of laccase CotA from *Bacillus subtilis* with indicated distances between the most important atoms (adopted from Enguita et al., "Crystal Structure of a Bacterial Endospore Coat Component", J. Biol. Chem., 278, 19416-19425, 2003). The area of MUT1 mutation is indicated by the dashed oval.

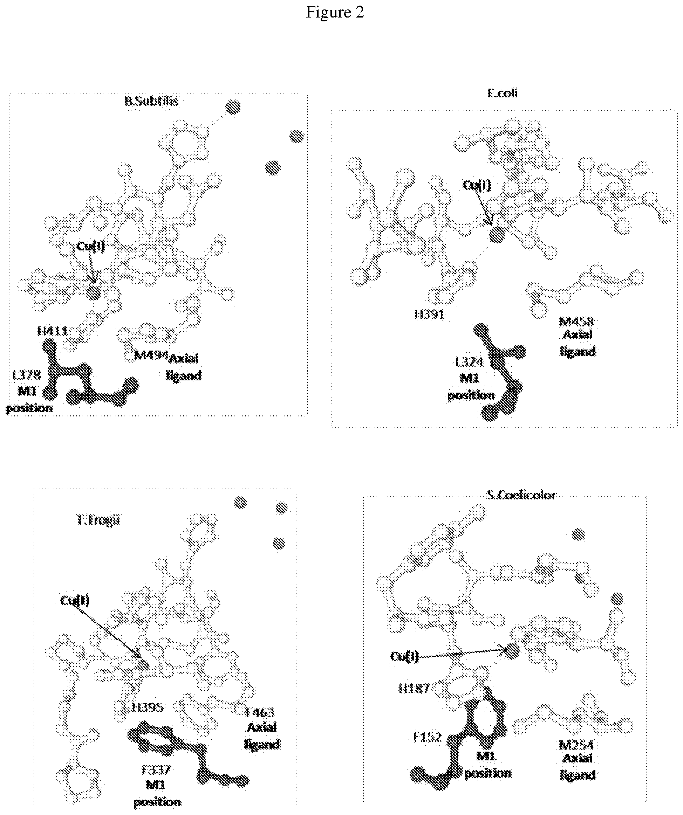

Figure 2 shows the three-dimensional structure of Cu1 site ligand environment in radius of 6A elucidated from crystal structures of four evolutionary distant laccase (*Bacillus Subtilis* COTA protein, *Streptomyces Coelicolor* laccase, *E.coli* CuEO laccase, and *Trametes Trogii* laccase). Respective accession numbers in Structure Data Base 1 UVW, 3KW8, 2FQD and 1KYA. Numeration of residues in *B.subtilis* laccase crystal structure is 9 residues less than that of the full size protein (a small N-terminal fragment was missing from the crystallized protein). A residue corresponding to the Glutamine 368 is depicted in black.

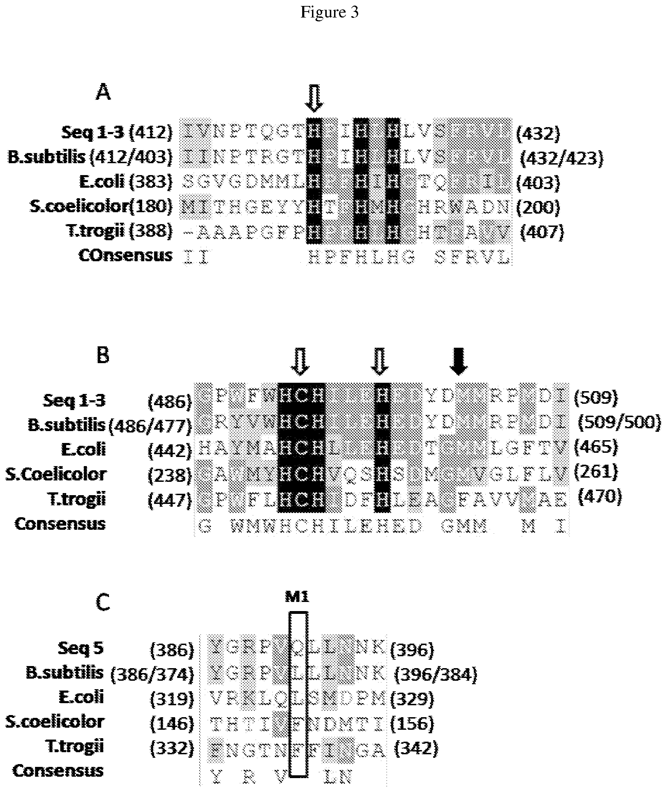

Figure 3 shows an alignment of the two conserved regions containing T1 copper ligands derived from evolutionary distant laccases (*Bacillus Subtilis* COTA protein, *Streptomyces Coelicolor* laccase, *E.coli* CuEO laccase, and *Trametes Trogii* laccase). Corresponding crystal structures of the Cu1 surrounding are presented in Fig. 2. Empty arrows indicate the positions of Cu-1 ligands, black arrow indicates axial ligand. Panel C shows just a list of the sequences surrounding Q386 substitution (M1) identified from the 3-D structures. M1 position is framed.

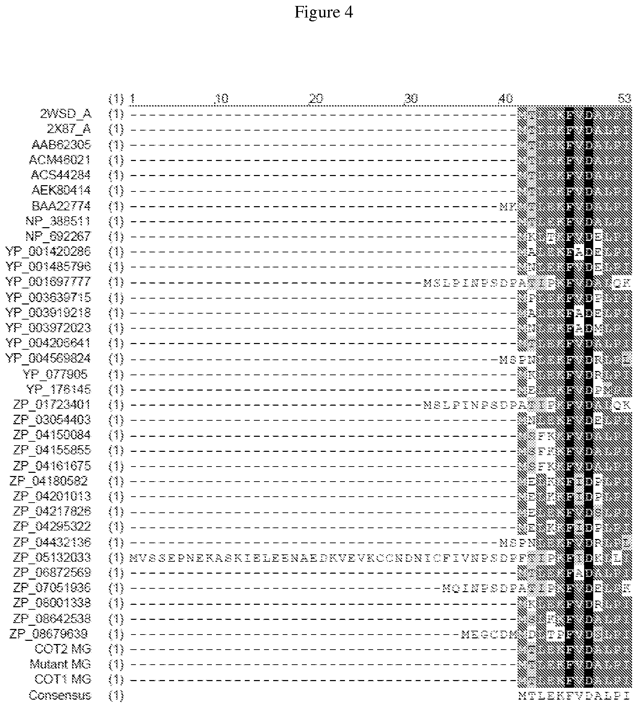

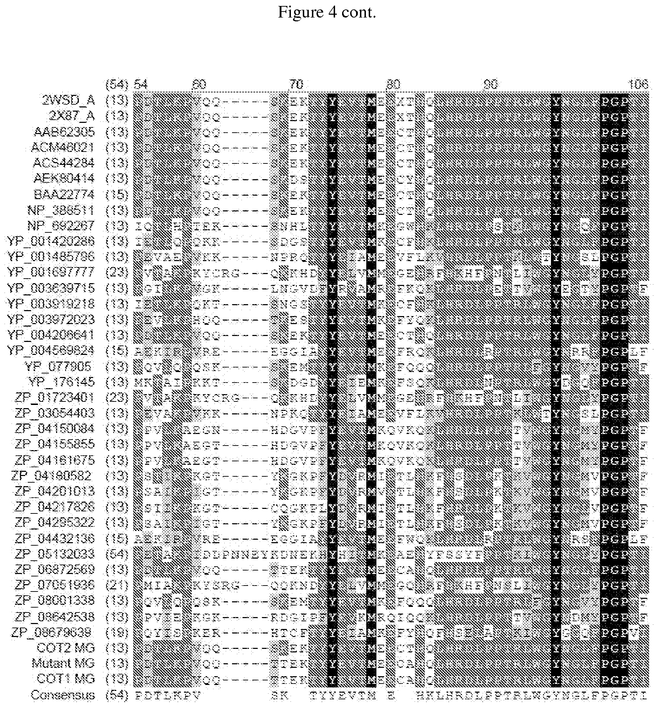

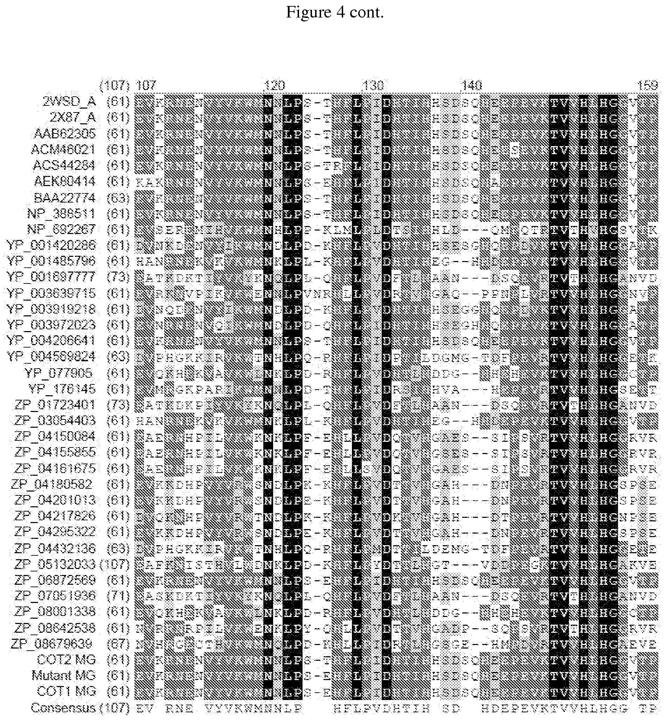

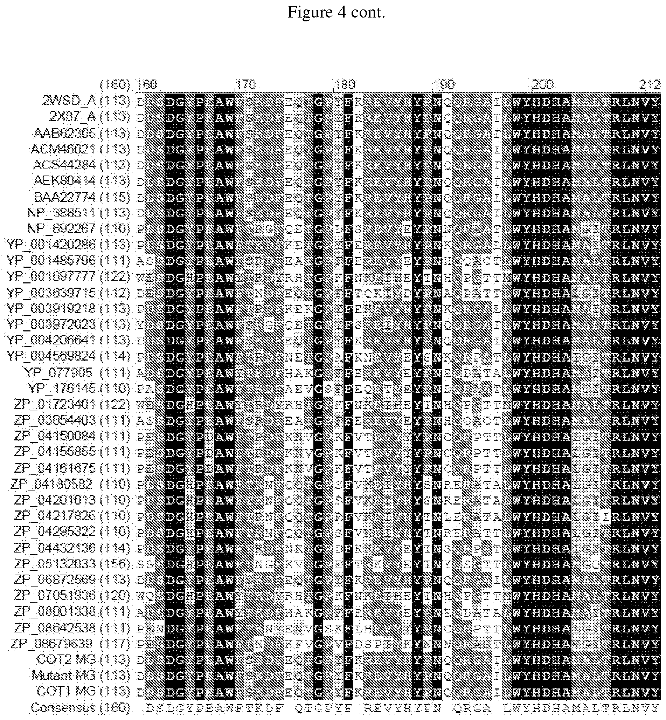

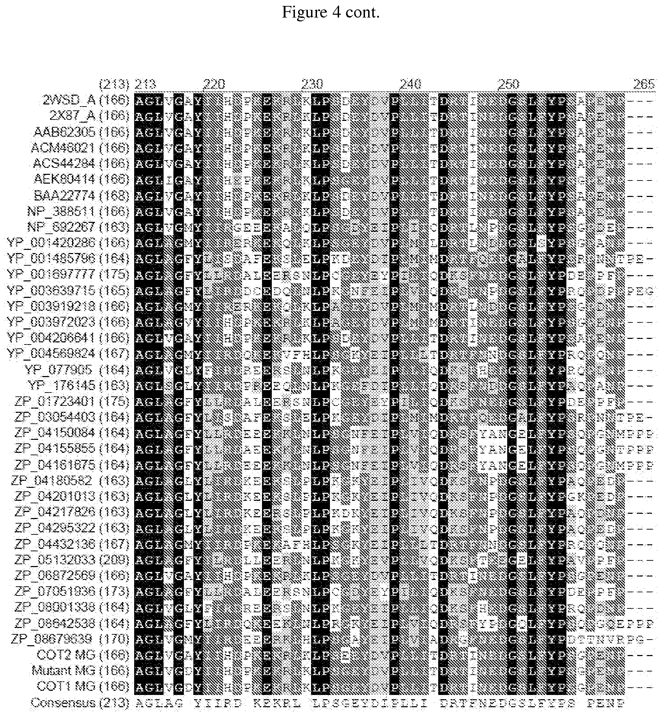

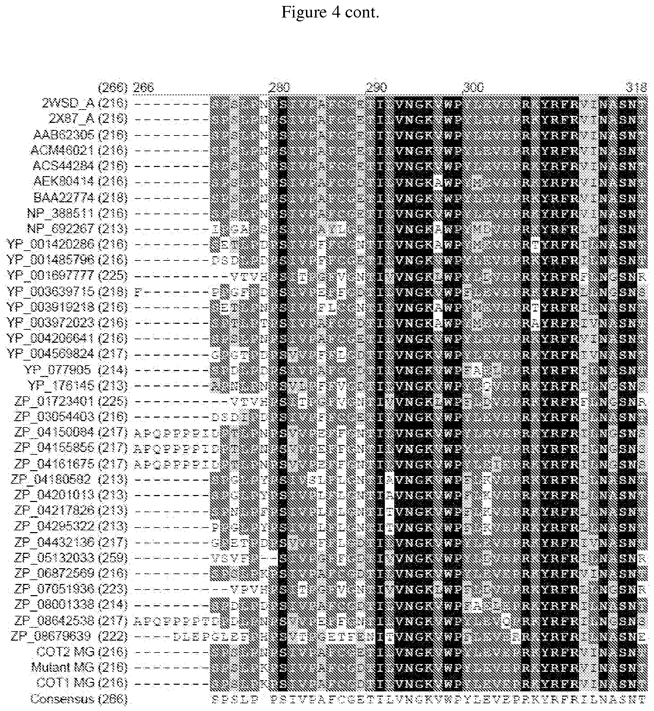

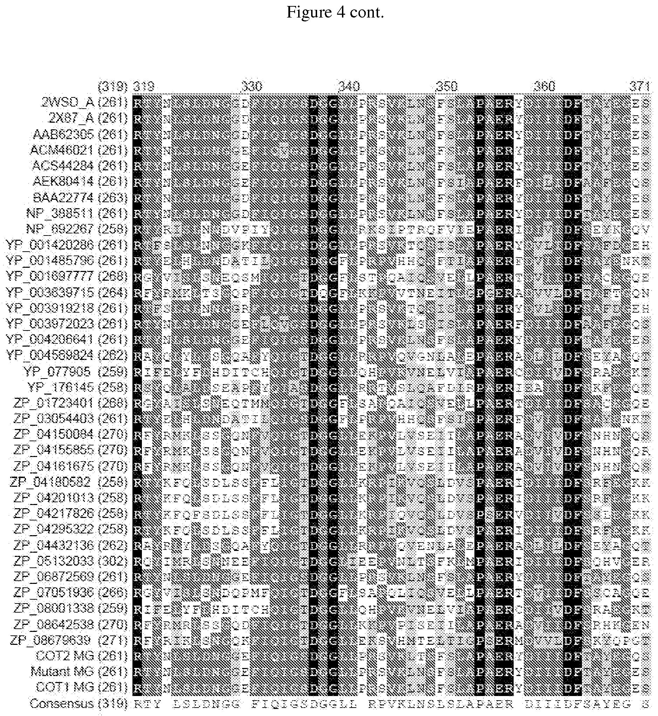

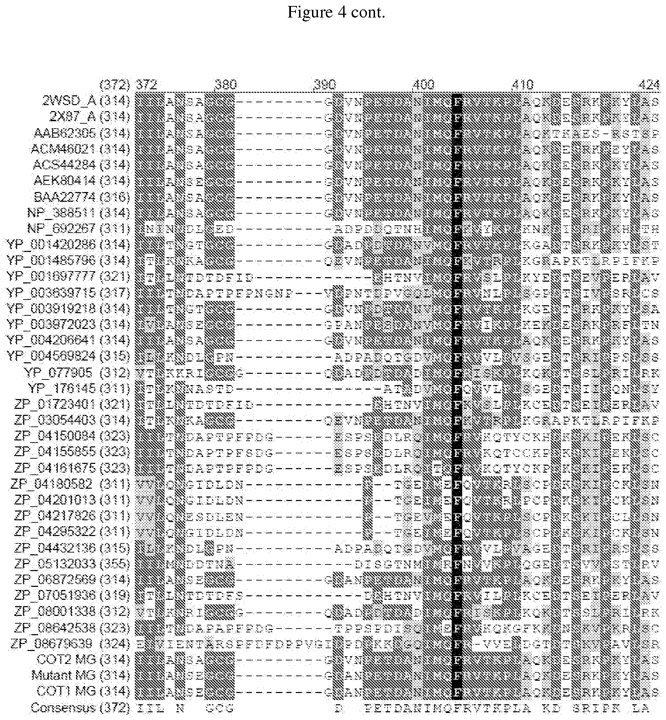

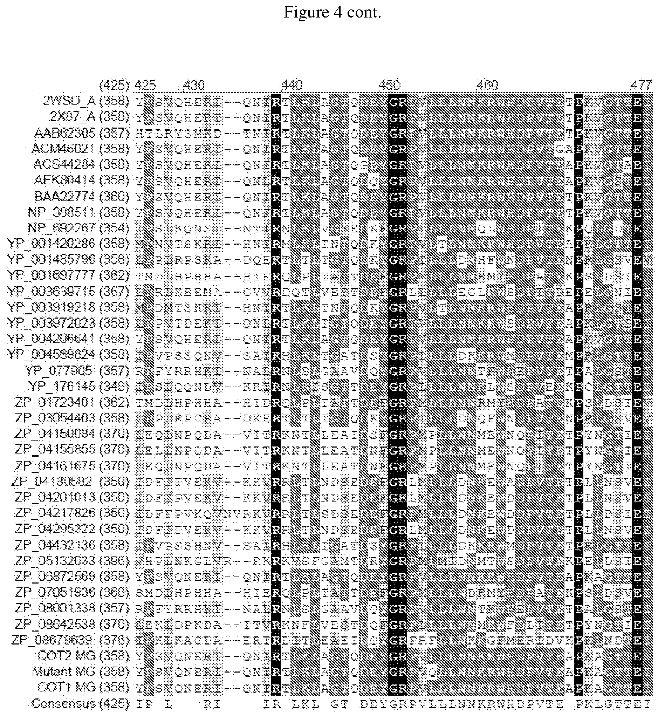

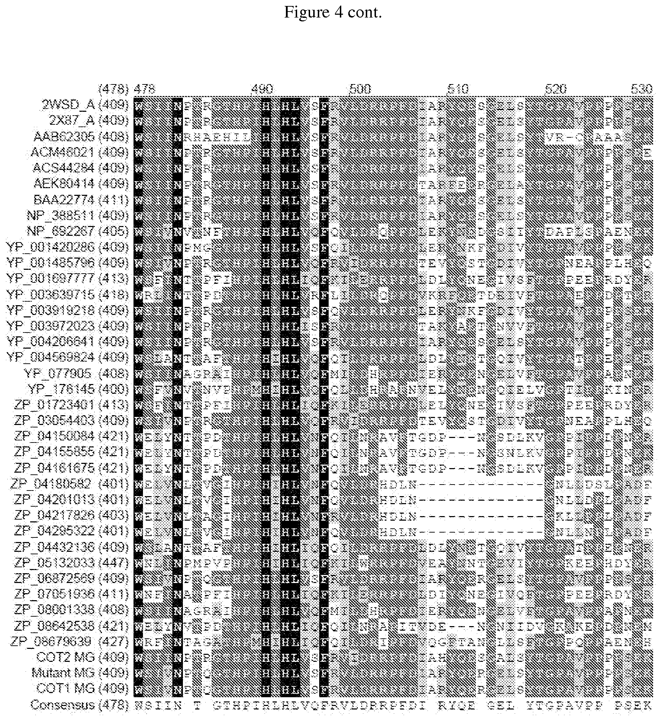

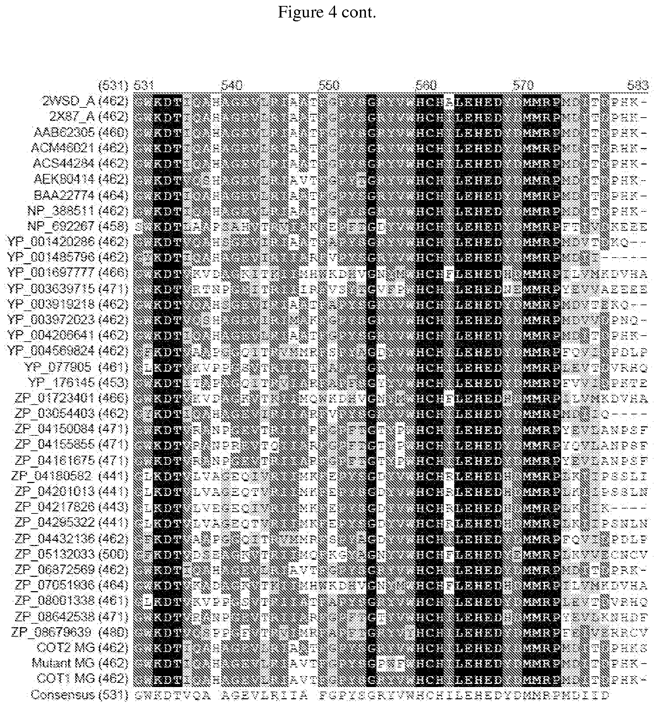

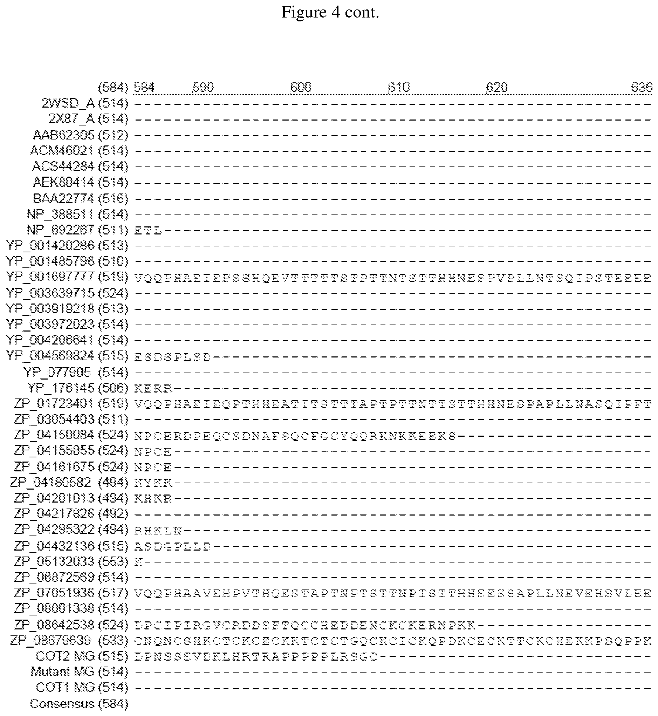

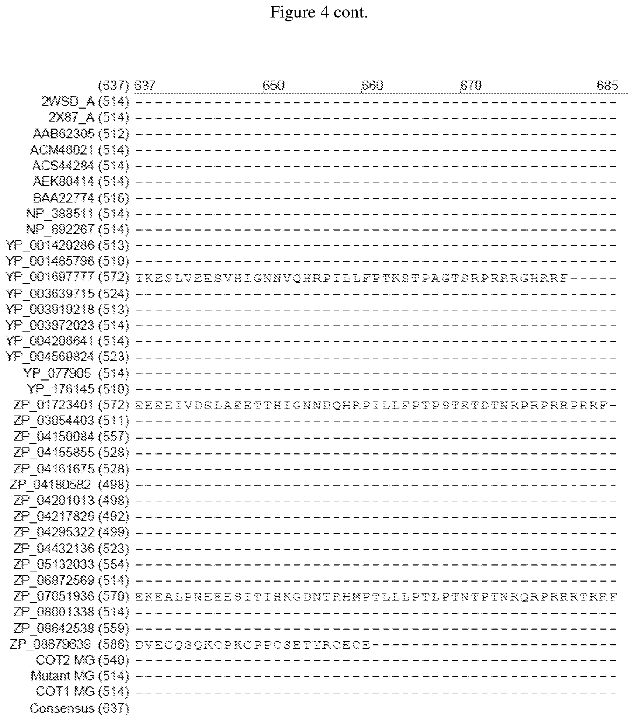

Figure 4 shows a multiple alignment of amino acid sequences that are related to COT1 (SEQ ID NO:1) and COT2 (SEQ ID NO:2) and were identified in a Blast search.

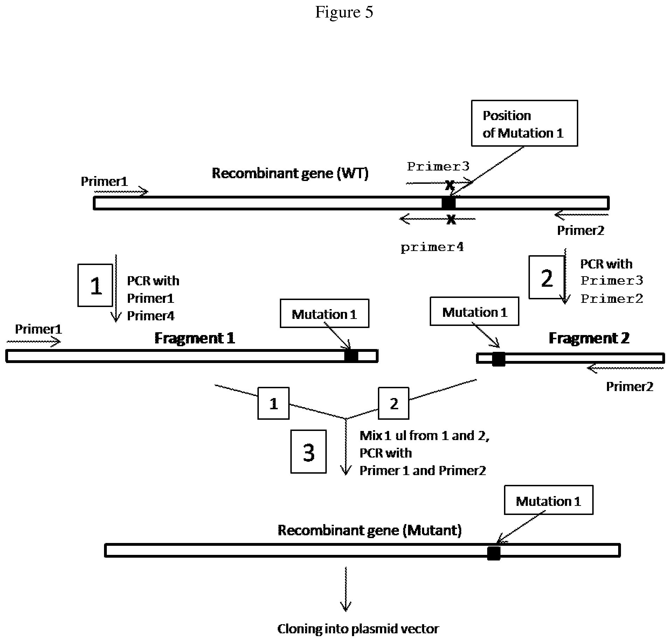

Figure 5 shows a schematic representation of introducing MUT1 into Laccase type2 gene from Bacillus pseudomycoides. Primers 1 and 2 represent terminal regions of the recombinant gene. Primers 3 and 4 represent fragments of the top and bottom strands of the mutated gene surrounding mutation site (X on the primers depicts the mutation). PCR reactions (1) and (2) produce two overlapping fragments of the gene (Fragment 1 and Fragment2), both bearing the mutation. The third PCR reassembles the full length gene with mutation (black bar) at the desired position.

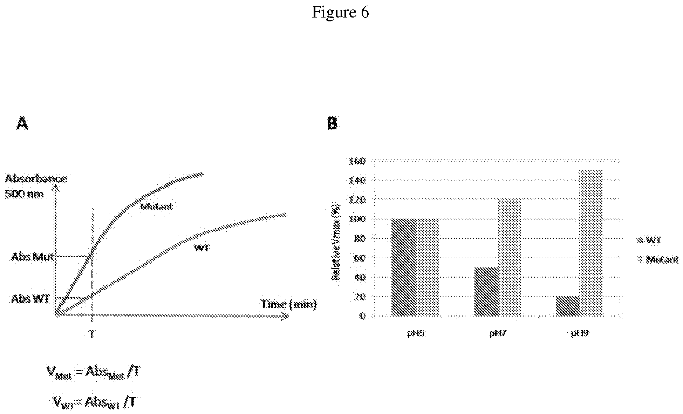

Figure 6 illustrates measurements of relative activity of the present laccases at different pH. Panel A demonstrates the selection of the initial rate time range for the reactions. As this time depends on the amount of the enzyme in the reaction, a suitable dilution of the enzyme needs to be obtained for convenient measurement. In the present examples, 10 min time was within the linear range in all pH conditions. Panel B illustrates the photometric measurement of ABTS absorbance; maximal initial rates of the present laccases (with and without mutation - WT and Mutant, respectively).

### DETAILED DESCRIPTION OF THE INVENTION

The present invention is based on a surprising finding that certain amino acid substitutions result in increased laccase activity especially in alkaline conditions.

The term "amino acid substitution" is used herein the same way as it is commonly used, i.e. the term refers to a replacement of one or more amino acids in a protein with another. Artificial amino acid substitutions may also be referred to as mutations.

As used herein, the term "alkaline" is a synonym for the term "basic". Thus, the term "alkaline conditions" refers to conditions having a pH value greater than 7.

The term "laccase activity" is used herein to mean maximal initial rate of the oxidation reaction. Laccase activity may be determined by standard oxidation assays known in the art including, but not limited to measurement of oxidation of Syringaldazine by laccase according to Sigma online protocol, or according to Cantarella et al. ("Determination of laccase activity in mixed solvents: Comparison between two chromogens in a spectrophotometric assay", Biotechnology and Bioengineering V. 82 (4), pp 395-398, 2003). An example of determining relative laccase activity at different pH is presented in Example 2. Any substrate suitable for the enzyme in question may be used in the activity measurements. A non-limiting example of a substrate suitable for use in assessing the enzymatic activity of the present laccase variants is 2,6-Dimethoxyphenol (2,6-DMP).

As used herein, the term "increased (or improved) laccase activity" refers to a laccase activity higher than that of a corresponding non-mutated laccase enzyme under the same conditions. That is to say, for instance if enzymes A and B have equal activity at pH5, whereas at pH 9 the same preparation of enzyme A has a higher activity than that of enzyme B, then enzyme A is denoted as a laccase variant having "an increased laccase activity in alkaline conditions". Certain amino acid variants in certain positions of laccase protein disclosed herein result in increased laccase activity at alkaline pH at least by 50% as compared to the corresponding laccase enzymes omitting this amino acid variant. In some embodiments, an increased laccase activity in alkaline conditions means about 2-fold, and preferably 5-fold, higher laccase activity as compared to that of a corresponding non-mutated variant.

Laccase molecules are usually monomers consisting of three consecutively connected cupredoxin-like domains twisted in a tight globule. The active site of laccases contains four copper ions: a mononuclear "blue" copper ion (T1 site) and a three-nuclear copper cluster (T2/T3 site) consisting of one T2 copper ion and two T3 copper ions (Fig. 1).

Laccases isolated from different sources are very diverse in primary sequences; however, they have some conserved regions in the sequences and certain common features in their three-dimensional structures. A comparison of sequences of more than 100 laccases has revealed four short conservative regions (no longer than 10 aa each) which are specific for all laccases (Kumar et al., "Combined sequence and structure analysis of the fungal laccase family", Biotechnol. Bioeng., 83, 386-394, 2003; Morozova et al., "Blue laccases", Biochemistry (Moscow), 72, 1136-1150, 2007). One cysteine and ten histidine residues form a ligand environment of copper ions of the laccase active site present in these four conservative amino acid sequences.

The T1 site of the enzyme is the primary acceptor of electrons from reducing substrates. The potential of the enzyme T1 site also determines the efficiency of catalysis on oxidation of the majority of laccase substrates, and therefore T1 site is primary target for laccase protein engineering. The T1 site has as ligands two histidine imidazoles and the sulfhydryl group of cysteine, which form a trigonal structure (Fig. 1). The fourth residue in the immediate proximity of the copper 1 is so called axial ligand - methionine or phenylalanine (Met502 in Fig. 1). These ligands in the primary sequence are situated in the two conserved regions (third and fourth) at the distal end of the protein.

Most residues forming the ligand environment of the type 1 copper ion are relatively conserved and three-dimensional structures of the copper binding domains from remotely related laccases may be very similar. As an example, Fig. 2 shows surrounding of copper 1 atom in 6 A radiuses of four evolutionary very distant laccases (sequence identity not more than 20%, length of the protein chain varies from 273 to 503 aa). All residues comprising copper 1 environment in these laccases are adjacent or proximal in the primary sequence to the copper ligands (two histidines and the cystein) and belong to the conserved regions, with one exception. A residue (marked dark in the Fig. 2, usually hydrophobic, in the depicted cases leucine or phenylalanine) is protruding into the environment of copper 1 atom from a distant part of the primary sequence.

It has now been surprisingly found that when the protruding amino acid is substituted by Glutamine (Q), the result is an improved laccase performance at alkaline conditions. This substitution is hereinafter referred to as MUT1, or M1.This position is situated in the part of the primary sequence which is not conserved between distant laccases. Fig. 3 shows fragments of aligned primary sequences of the laccases from Fig. 2. All residues depicted in the crystal structures in fair grey are situated in the regions depicted in panels A and B (conserved regions). Whereas the residue depicted in crystal structures black (Fig. 2, M1 position) is situated in the regions depicted on panel C. Panel C was not generated by alignment protocols owing to lack of sufficient homology in these part of the sequences), but the panel is only a list of sequences surrounding the MUT1 position elucidated from 3-D structure (marked black in Fig. 2).

In other examples where laccases in question are more homologous, this region may be sequentially conserved, and thus MUT1 position may be elucidated from a sequence alignment. Whether sequentially conserved or not, this residue can be unambiguously identified in practically any laccase by being present in an about 5-6 A radius of copper 1 in proximity to the axial ligand of Copper 1 atom. To our best knowledge there is no glutamine in the copper-1 5-6 A environment in any of the laccases with a known three-dimensional structure.

In connection with the present invention, two laccase protein sequences COT1 (SEQ ID NO:1) and COT2 (SEQ ID NO:2) absent from publicly available databases were cloned from laboratory strains of *Bacillus subtilis.* In-silica analysis of the protein structures together with intensive experimental research using combinatorial methods of molecular biology confirmed that an artificial Leucine (L) to Glutamine (Q) substitution in position 386 of both SEQ ID NO:1 and SEQ ID NO:2 improved laccase performance at alkaline conditions. Improved performance was also achieved by another mutation, i.e. adjacent Arginine (R) 487 to Proline (P), Tyrosine (Y) 488 to Tryptophan (W) and Valine (V) 489 to Phenylalanine (F) triple substitution, either alone or in combination with the L386Q substitution. Amino acid sequence of a laccase variant comprising both of these mutations is depicted in SEQ ID NO:3, whereas amino acid sequences depicted in SEQ ID NO: 4 and SEQ ID NO:5 represent laccase variants comprising only the L386Q substitution or the triple substitution, respectively.

Amino acid variants presented by these mutations appear to be unique at corresponding positions among related polypeptide sequences since they were not identified in a protein search in BLAST, a public internet service which compares the query sequence to all sequences deposited in the public domain. The search revealed some closely related sequences only a few amino acids different from the queries and a whole range of homologous sequences with different degree of similarity (Table 1).

**Table 1. The results of the Blast search**

| The sequences (accession numbers) are listed in the order of decreasing similarity. | | | | |
|---|---|---|---|---|
| **Accession** | **Description** | **Identity %** | **Similarity %** | **M1-3ple Q...PWF** |
| YP_004206641.1 | spore copper-dependent laccase [Bacillus subtilis BSn5] bj\|BAI84141.1\| spore coat protein A [Bacillus subtilis subsp. natto BEST195] >gb\|ADV95614.1\| spore copper-dependent laccase [Bacillus subtilis BSn5] spore copper-dependent laccase [Bacillus subtilis subsp. subtilis str. 168] >ref\|ZP_03590314.1\| spore coat protein (outer) [Bacillus subtilis subsp. subtilis str. 168] >ref\|ZP_03594593.1\| spore coat protein (outer) [Bacillus subtilis subsp. subtilis str. NCIB 3610] >ref\|ZP_03599005.1\| spore coat protein (outer) [Bacillus subtilis subsp. subtilis str. JH642] >ref\|ZP_03603283.1\| spore coat protein (outer) [Bacillus subtilis subsp. subtilis str. SMY] >sp\|P07788.4\|COTA_BACSU RecName: Full=Spore coat protein A >pdb\|1GSK\|A Chain A, Crystal Structure Of Cota, An | 98 | 99 | L...RYV |
| NP_388511.1 | Endospore Coat Protein From Bacillus Subtilis >pdb\|1OF0\|A Chain A, Crystal Structure Of Bacillus Subtilis Cota After 1h Soaking With Ebs >pdb\|1UVW\|A Chain A, Bacillus Subtilis Cota Laccase Adduct With Abts >pdb\|1W6L\|A Chain A, 3d Structure Of Cota Incubated With Cucl2 >pdb\|1W6W\|A Chain A, 3d Structure Of Cota Incubated With Sodium Azide >pdb\|1W8E\|A Chain A, 3d Structure Of Cota Incubated With Hydrogen Peroxide >pdb\|2BHF\|A Chain A, 3d Structure Of The Reduced Form Of Cota >pdb\|2X88\|A Chain A, Crystal Structure Of Holocota >emb\|CAB12449.1\| spore copper-dependent laccase [Bacillus subtilis subsp. subtilis str. 168] | 98 | 99 | L...RYV |
| BAA22774.1 | spore coat proein A [Bacillus subtilis] | 98 | 99 | L...RYV |
| ACS44284.1 | spore coat protein [Bacillus subtilis] | 98 | 98 | L...RYV |
| 2X87_A | Chain A, Crystal Structure Of The Reconstituted Cota | 98 | 99 | L...RYV |
| 2WSD_A | Chain A, Proximal Mutations At The Type 1 Cu Site Of Cota-Laccase: I494a Mutant | 97 | 98 | L...RYV |
| ACM46021.1 | laccase [Bacillus sp. HR03] spore copper-dependent laccase [Bacillus subtilis subsp. spizizenii ATCC 6633] >ref\|YP_003865004.1\| spore copper-dependent laccase (outer coat) [Bacillus subtilis subsp. | 97 | 98 | L...RYV |
| ZP_06872569.1 | spizizenii str. W23] >gb\|EFG93543.1\| spore copper-dependent 1031 laccase [Bacillus subtilis subsp. spizizenii ATCC 6633] >gb\|ADM36695.1\| spore copper-dependent laccase (outer coat) [Bacillus subtilis subsp. spizizenii str. W23] | 96 | 97 | L...RYV |
| AAB62305.1 | CotA [Bacillus subtilis] spore copper-dependent laccase [Bacillus atrophaeus 1942] | 91 | 94 | L...RYV |
| YP_003972023.1 | >gb\|ADP31092.1\| spore copper-dependent laccase (outer coat) [Bacillus atrophaeus 1942] spore copper-dependent laccase [Bacillus amyloliquefaciens DSM 7] >emb\|CBI41748.1\| spore copper-dependent laccase [Bacillus amyloliquefaciens DSM 7] >gb\|AEB22768.1\| spore | 82 | 91 | L...RYV |
| YP_003919218.1 | copper-dependent laccase [Bacillus amyloliquefaciens TA208] >gb\|AEB62213.1\| spore copper-dependent laccase [Bacillus amyloliquefaciens LL3] >gb\|AEK87755.1\| spore copper-dependent laccase [Bacillus amyloliquefaciens XH7] | 77 | 89 | L...RYV |
| YP_001420286.1 | CotA [Bacillus amyloliquefaciens FZB42] >gb\|ABS73055.1\| CotA Bacillus amyloliquefaciens FZB42] spore coat protein A [Bacillus pumilus ATCC 7061] | 77 | 89 | L...RYV |
| ZP_03054403.1 | >gb\|EDW21710.1\| spore coat protein A [Bacillus pumilus ATCC 7061] | 69 | 79 | L...RYV |
| YP_001485796.1 | outer spore coat protein A [Bacillus pumilus SAFR-032] >gb\|ABV61236.1\| outer spore coat protein A [Bacillus pumilus SAFR-032] | 68 | 79 | L...RYV |
| ZP_08001338.1 | CotA protein [Bacillus sp. BT1B_CT2] >gb\|EFV71562.1\| CotA protein [Bacillus sp. BT1B_CT2] | 65 | 77 | L...RYV |
| *YP_077905.1* | *spore coat protein [Bacillus licheniformis ATCC 14580]* >*ref*\|*YP_090310.1*\| *CotA [Bacillus licheniformis ATCC 14580]* >*gb*\|*AAU22267.1*\| *spore coat protein (outer) [Bacillus licheniformis ATCC 14580]* >*gb*\|*AAU39617.1*\| *CotA [Bacillus licheniformis ATCC 14580]* | 65 | 77 | *L...RYV* |
| NP_692267.1 | spore outer coat protein [Oceanobacillus iheyensis HTE831] >dbj\|BAC13302.1\| spore coat protein (outer) [Oceanobacillus iheyensis HTE831] | 60 | 74 | L...DYV |
| YP_176145.1 | spore coat protein [Bacillus clausii KSM-K16] >dbj\|BAD65184.1\| spore coat protein [Bacillus clausii KSM-K16] | 59 | 75 | L...YYV |
| ZP_04432136.1 | Bilirubin oxidase [Bacillus coagulans 36D1] >gb\|EEN93171.1\| Bilirubin oxidase [Bacillus coagulans 36D1] | 60 | 74 | L...DYV |
| YP_004569824.1 | Bilirubin oxidase [Bacillus coagulans 2-6] >gb\|AEH54438.1\| Bilirubin oxidase [Bacillus coagulans 2-6] | 59 | 73 | L...DYV |
| ZP_04217826.1 | Multicopper oxidase, type 2 [Bacillus cereus Rock3-44] >gb\|EEL50489.1\| Multicopper oxidase, type 2 [Bacillus cereus Rock3-44] | 52 | 71 | L...DYV |
| ZP_04295322.1 | Multicopper oxidase, type 2 [Bacillus cereus AH621] >gb\|EEK73233.1\| Multicopper oxidase, type 2 [Bacillus cereus AH621] | 53 | 70 | L...DYV |
| ZP_04201013.1 | Spore coat protein A [Bacillus cereus AH603] >gb\|EEL67287.1\| Spore coat protein A [Bacillus cereus AH603] | 53 | 70 | L...DYV |
| ZP_04180582.1 | Spore coat protein A [Bacillus cereus AH1272] >gb\|EEL87731.1\| Spore coat protein A [Bacillus cereus AH 1272] | 53 | 70 | L...DYV |
| ZP_04150084.1 | Multicopper oxidase, type 2 [Bacillus pseudomycoides DSM 12442] >gb\|EEM18231.1\| Multicopper oxidase, type 2 [Bacillus pseudomycoides DSM 12442] | 51 | 67 | L...TYP |
| YP_003639715.1 | Bilirubin oxidase [Thermincola sp. JR] >gb\|ADG81814.1\| Bilirubin oxidase [Thermincola potens JR] | 53 | 68 | L...VFP |
| ZP_04155855.1 | Multicopper oxidase, type 2 [Bacillus mycoides Rock3-17] >gb\|EEM12426.1\| Multicopper oxidase, type 2 [Bacillus mycoides Rock3-17] | 52 | 67 | L...TYP |
| ZP_04161675.1 | Multicopper oxidase, type 2 [Bacillus mycoides Rock1-4] >gb\|EEM06612.1\| Multicopper oxidase, type 2 [Bacillus mycoides Rock1-4] | 52 | 67 | L...TYP |
| ZP_08642538.1 | spore coat protein A [Brevibacillus laterosporus LMG 15441] >gb\|EGP32769.1\| spore coat protein A [Brevibacillus laterosporus LMG 15441] | 51 | 68 | L...TYV |
| ZP_08679639.1 | spore coat protein A [Sporosarcina newyorkensis 2681] >gb\|EGQ24147.1\| spore coat protein A [Sporosarcina newyorkensis 2681] | 50 | 65 | L...RYV |
| YP_001697777.1 | spore coat protein A [Lysinibacillus sphaericus C3-41] >gb\|ACA39647.1\| Spore coat protein A [Lysinibacillus sphaericus C3-41] | 52 | 67 | L...NYM |
| ZP_05132033.1 | spore coat protein [Clostridium sp. 7_2_43FAA] >gb\|EEH98927.1\| spore coat protein [Clostridium sp.7_2_43FAA] | 51 | 65 | L...NYV |
| ZP_01723401.1 | spore coat protein (outer) [Bacillus sp. B14905] >gb\|EAZ86095.1\| spore coat protein (outer) B14905] | 52 | 66 | L...NYM |
| ZP_07051936.1 | spore coat protein A [Lysinibacillus fusiformis ZC1]>gb\|EFI66832.1\| spore coat protein A [Lysinibacillus fusiformisZC1] | 50 | 66 | L...NYM |

In order to create a more general picture of the structure of the related sequences, multiple alignments of the revealed sequences were performed. Over 30 most similar sequences ranging from 98 to 50% identity to the query sequences were downloaded to VectorNTI® software (Invitrogen) and arranged in a multiple alignment in the same order as in the BLAST list (Figure 4). The alignment confirmed the uniqueness of the present amino acid substitutions.

Mutations corresponding to the Q386 mutation and/or P487/W488/F489 triple mutation shown in SEQ ID NO:3 may be introduced to any of the amino acid sequences disclosed herein, or other homologous sequences, by standard methods known in the art, such as site-directed mutagenesis, in order to improve their laccase activity in alkaline conditions. Kits for performing site-directed mutagenesis are commercially available in the art (e.g. QuikChange® II XL Site-Directed Mutagenesis kit by Agilent Technologies). Further suitable methods for introducing the above mutations into a recombinant gene are disclosed e.g. in Methods in Molecular Biology, Vol 182, "In vitro mutagenesis protocols", Eds Jeff Braman, Humana Press 2002). Thus, provided herein are laccase variants or mutants which comprise Glutamine (Q) in a position which corresponds to the position 386 of the amino acid sequence depicted in SEQ ID NO:3 (denoted as MUT1) optionally also comprising a Proline-Tryptophan-Phenylalanine (PWF) triple mutation in a position which corresponds to the position 487-489 of the amino acid sequence depicted in SEQ ID NO:3 (MUT2), and have an increased laccase activity in alkaline conditions as compared to that of a corresponding non-mutated control variant (Table 2).

**Table 2. Effect of mutations MUT1 and the triple mutation (MUT2) on relative activities of laccase proteins at different pH. All mutations were beneficial for activity even at acidic pH; however a much larger effect was observed at elevated pH values.**

| | % act at pH5 | % act at pH7 | % act at pH9 |
|---|---|---|---|
| SEQ ID NO:1 | 100 | 60 | 23 |
| SEQ ID NO:1 +MUT1 | 140 | 170 | 220 |
| SEQ ID NO:1 + MUT2 | 120 | 130 | 150 |
| SEQ ID NO:1 + MUT1 + MUT2 | 180 | 210 | 300 |
| SEQ ID NO:2 | 100 | 60 | 25 |
| SEQ ID NO:2 + MUT1 | 130 | 160 | 200 |
| SEQ ID NO:2 + MUT 2l | 120 | 130 | 150 |
| SEQ ID NO:2 + MUT 1 + MUT2 | 160 | 200 | 300 |

Amino acid sequences revealed in the Blast search may be represented as a consensus sequence. SEQ ID NO:6 represent a consensus sequence of 33 amino acid sequences most closely related to the COT1 and COT2 query sequences. Thus, described herein are laccase variants comprising an amino acid sequence depicted in SEQ ID NO:6 introduced with a MUT1 and/or MUT2 mutation.

Also described herein are laccase variants, i.e. homologues, having an increased enzyme activity in alkaline conditions that comprise an amino acid sequence which has at least 50% sequence identity with the variants comprising an amino acid sequence selected from the group consisting of SEQ ID NO:3 (comprising MUT1 + MUT2); SEQ ID NO:4 (comprising MUT1), and SEQ ID NO:5 (comprising MUT2). Also disclosed herein are variants wherein said amino acid sequence is selected from a group consisting of SEQ ID NO:1, SEQ ID NO:2, SEQ ID NO:6, and any of the sequences shown in Figure 4, further comprising a mutation corresponding to MUT1 and/or MUT2. Also described herein are laccase variants which comprise an amino acid sequence having a degree of identity to any of the above-mentioned reference sequences of at least about 55%, preferably about 65%, more preferably about 75%, still more preferably about 85%, and even more preferably about 95%, 96%, 97%, 98%, or 99%, and which retain increased laccase activity in alkaline conditions. In some variants, the degree of identity corresponds to any value between the above-mentioned integers.

As used herein, the degree of identity between two or more amino acid sequences is equivalent to a function of the number of identical positions shared by the sequences (i.e., % identity = # of identical positions/total # of positions x 100), excluding gaps, which need to be introduced for optimal alignment of the two sequences, and overhangs. The comparison of sequences and determination of percent identity between two or more sequences can be accomplished using standard methods known in the art.

The present laccase variants may comprise conservative amino acid substitutions as compared to any of the sequences depicted in SEQ ID NO:1, SEQ ID NO:2, SEQ ID NO:3, SEQ ID NO:4, SEQ ID NO:5, SEQ ID NO:6, and Figure 4. The term "conservative amino acid substitution" refers to a replacement of an amino acid with a similar amino acid as known in the art. Conservative amino acid substitutions do not significantly affect the folding and/or activity of a protein sequence variant. Typical non-limiting examples of such conservative amino acid substitutions include substitution of glutamate for aspartate or vice versa.

The present laccase variants may further comprise amino acid deletions and/or additions as long as they retain their increased laccase activity in alkaline conditions. In this context, the term "functional fragment" refers to a truncated laccase polypeptide retaining said increased enzyme activity in alkaline conditions.

As used herein, the term "conservative variant" refers to polypeptides comprising conservative amino acid substitutions, deletions and/or additions, and retaining their enzymatic properties, especially increased laccase activity in alkaline conditions.

The present laccase polypeptides or proteins may be fused to additional sequences, by attaching or inserting, including , but not limited to, affinity tags, facilitating protein purification (S-tag, maltose binding domain, chtin binding domain), domains or sequences assisting folding (such as thioredoxin domain, SUMO protein), sequences affecting protein localization (periplasmic localization signals etc), proteins bearing additional function, such as green fluorescent protein (GFP), or sequences representing another enzymatic activity. Other suitable fusion partners for the present laccases are known to those skilled in the art.

The present disclosure also provides isolated polynucleotides encoding any of the laccase variants disclosed herein. Means and methods for cloning and isolating such polynucleotides are well known in the art.

Furthermore, the present disclosure also provides vectors comprising the present polynucleotides operably linked to one or more control sequences. Suitable control sequences are readily available in the art and include, but are not limited to, promoter, leader, polyadenylation, and signal sequences.

Laccase variants according to various embodiments of the present invention may be obtained by standard recombinant methods known in the art. Briefly, such a method may comprise the steps of i) culturing a desired recombinant host cell under conditions suitable for the production of a present laccase polypeptide variant, and ii) recovering the polypeptide variant obtained. A large number of vector-host systems known in the art may be used for recombinant production of laccase variants. Possible vectors include, but are not limited to, plasmids or modified viruses which are maintained in the host cell as autonomous DNA molecule or integrated in genomic DNA. The vector system must be compatible with the host cell used as well known in the art. Non-limiting examples of suitable host cells include bacteria (e.g. *E.coli,* bacilli), yeast (e.g. Pichia Pastoris, Saccharomyces Cerevisae), fungi (e.g. filamentous fungi) insect cells (e.g. Sf9).

Recovery of a laccase variant produced by a host cell may be performed by any technique known to those skilled in the art. Possible techniques include, but are not limited to secretion of the protein into the expression medium, and purification of the protein from cellular biomass.

The production method may further comprise a step of purifying the laccase variant obtained. For thermostable laccases, non-limiting examples of such methods include heating of the disintegrated cells and removing coagulated thermo labile proteins from the solution. For secreted proteins, non-limiting examples of such methods include ion exchange chromatography, and ultra-filtration of the expression medium. It is important that the purification method of choice is such that the purified protein retains its laccase activity.

The present laccase variants may be used in a wide range of different industrial processes and applications, such as in pulp delignification, textile dye bleaching, wastewater detoxifixation, xenobiotic detoxification, and detergent manufacturing. The increased operable pH range of the disclosed laccase variants makes them particularly suitable for industrial waste water treatment processes.

It will be obvious to a person skilled in the art that, as the technology advances, the inventive concept can be implemented in various ways. The invention and its embodiments are not limited to the examples described below but may vary within the scope of the claims.

### Example 1. Construction of laccase variants bearing MUT1

Mutations as described herein were introduced into various recombinant genes by standard site-directed mutagenesis. For instance, MUT1 (L386Q substitution) was introduced into the gene of Multicopper oxidase, type 2 from Bacillus pseudomycoides (ZP_04150084), which has approximately 50% sequence identity to the COT1 (SEQ ID NO:1) and COT2 (SEQ ID NO.2) laccases, by PCR amplifying the coding sequence of this gene (accession number NZ_ACMX01000022) from genomic DNA of Bacillus pseudomycoides and cloning it into a pET20 plasmid vector.

To this end, two series of separate PCR reactions were carried out: (1) with Primer1 (5'-CGCCGTCTCACATGTCTTTTAAAAAATTTGTC-GATGCATTACC-3'; SEQ ID NO: 7) and Primer4 (5'-ATAGTT-TTGGACGCCCTATGCCATTATTAAATAACATGG-AGT-3'; SEQ ID NO: 8), and (2) with Primer2 (5'-CGCGGATCCGATGATTTCTCTTCTTTTTTATTTTT-CCGTTG-3'; SEQ ID NO:9) and Primer3 (5'-ACTCCA-TGTTATTTAATAA-TGGCATAGGGCGTCCAAAACTAT-3'; SEQ ID NO.10).

In both PCR series, recombinant wild type gene was used as the template. Aliquots of 1 µl from reactions (1) and (2) were combined and used as template for PCR reaction with Primer 1 and Primer 2 (see above). Product of this reaction containing the mutant sequence of the gene was cloned in a plasmid vector for expression in *E.coli.* Schematic representation of this mutagenesis strategy is presented in Figure 5.

### Example 2. Relative activity measurement of laccase variants at different pH using 2,6-DMP

In the present experiments, 2,6-Dimethoxyphenol (2,6-DMP), which can be oxidized by wild type COT1 and COT2 laccases readily at pH 5 but much more slowly at pH 9, was chosen as the substrate.

Two forms of each enzyme - one possessing the mutation (Mut) and one without the mutation (further called wild type, WT) was tested in 2,6-Dimethoxyphenol (2,6-DMP) oxidation reactions at various pH. Reaction course was monitored by Absorbance at 500 nM.

Initial rates of the reactions were measured in OD(500)/min. Initial rate (V) is velocity of the reaction in the time range when the colour develops linearly with time. Similar reactions were carried out at different substrate (2,6-DMP) concentrations (see protocol below). Then maximum initial rate (Vmax) was determined at each pH (this rate was observed at saturating substrate concentrations).

In order to determine relative alkaline activity, for each enzyme its Vmax at pH5 was taken for 100%, and relative activity at pH7 or pH9 was determined as a fraction of this activity.

As an example, 2,6-DMP concentration of 0.5 mM was saturating for both WT and MUT enzymes at pH5 through pH9. MOPS buffer (3-(N-Morpholino) propane sulfonic acid, Sigma) was used as a reaction medium. Vmax of these two enzymes were determined according to the protocol:

| | |
|---|---|
| • MOPS 100 mM pH (5-9) | 90 µl, |
| • 2,6-DMP 5 mM | 10 µl, |
| Laccase (WT or MUT) | 2 µl, |

Incubation 10 min at 60 C

Absorbance at 500 nm was measured by titer plate reader.

As demonstrated in Figure 6, introducing MUT1 into the laccase polypeptide increases its relative activity at pH9 approximately 7-fold as compared to the non-mutated enzyme.

As well known to a person skilled in the art, the relative laccase activity at different pHs may be measured by any other substrate suitable for the laccase variant in question as long as the other substrate cab be oxidized at the same pH range (preferably pH 5 to pH 9). Also other parameters such as temperature may be adjusted to the particular laccase variant in question.

## Claims

1. A polypeptide having laccase activity comprising an amino acid sequence showing at least 50 % identity to an amino acid sequence selected from the group consisting of SEQ ID NO:3, SEQ ID NO:4, and SEQ ID NO:5 wherein a glutamine residue is in a position which corresponds to the position 386 of the amino acid sequence according to SEQ ID NO:3.

2. The polypeptide according to claim 1 additionally comprising a Proline-Tryptophan-Phenylalanine (PWF) sequence in a position which corresponds to the position 487-489 of the amino acid sequence according to SEQ ID NO:3.

3. The polypeptide according to claim 1 or 2, comprising an amino acid sequence selected from the group consisting of SEQ ID NO:1 and SEQ ID NO:2, comprising at least one amino acid variant selected from the group consisting of glutamine (Q) in a position which corresponds to the position 386 of the amino acid sequence according to SEQ ID NO:3 and a Proline-Tryptophan-Phenylalanine (PWF) sequence in a position which corresponds to the position 487-489 of the amino acid sequence according to SEQ ID NO:3.

4. A nucleic acid molecule encoding a polypeptide according to any one of claims 1 to 3.

5. A vector comprising a nucleic acid molecule according to claim 4.

6. A recombinant host cell comprising a vector according to claim 5.

7. A method of producing a polypeptide according to any one of claims 1 to 3, comprising the steps of:
i. culturing a recombinant host cell according to claim 6 under conditions suitable for the production of the polypeptide, and
ii. recovering the polypeptide obtained.

8. The method according to claim 7 further comprising a step of purifying said polypeptide.

9. Use of a polypeptide according to any one of claims 1 to 3 in pulp delignification.

10. Use of a polypeptide according to any one of claims 1 to 3 in textile dye bleaching.

11. Use of a polypeptide according to any one of claims 1 to 3 in wastewater detoxifixation.

12. Use of a polypeptide according to any one of claims 1 to 3 in xenobiotic detoxification.

13. A method for increasing the laccase activity of a polypeptide comprising an amino acid sequence showing at least 50 % identity to an amino acid sequence selected from the group consisting of SEQ ID NO:3, SEQ ID NO:4, and SEQ ID NO:5 wherein the amino acid which corresponds to the position 386 of the amino acid sequence according to SEQ ID NO:3, is replaced by a glutamine residue.

14. The method according to claim 13 wherein the three amino acids which correspond to the positions 487-489 of the amino acid sequence according to SEQ ID NO:3 are replaced with a Proline-Tryptophan-Phenylalanine (PWF) sequence.

## Patentansprüche

1. Polypeptid mit Laccase-Aktivität, umfassend eine Aminosäuresequenz, die mindestens 50% Identität zu einer Aminosäuresequenz aufweist, die aus der aus SEQ ID NO: 3, SEQ ID NO: 4 und SEQ ID NO: 5 bestehenden Gruppe ausgewählt ist, wobei sich ein Glutaminrest in einer Position befindet, die der Position 386 der Aminosäuresequenz gemäß SEQ ID NO: 3 entspricht.

2. Polypeptid nach Anspruch 1, das zudem eine Prolin-Tryptophan-Phenylalanin-(PWF)-Sequenz in einer Position umfasst, die der Position 487-489 der Aminosäuresequenz gemäß SEQ ID NO: 3 entspricht.

3. Polypeptid nach Anspruch 1 oder 2, umfassend eine Aminosäuresequenz, die aus der aus SEQ ID NO: 1 und SEQ ID NO: 2 bestehenden Gruppe ausgewählt ist, umfassend mindestens eine Aminosäurevariante, die aus der aus Glutamin (Q) in einer Position, die der Position 386 der Aminosäuresequenz gemäß SEQ ID NO: 3 entspricht, und einer Prolin-Tryptophan-Phenylalanin-(PWF)-Sequenz in einer Position, die der Position 487-489 der Aminosäuresequenz gemäß SEQ ID NO: 3 entspricht, bestehenden Gruppe ausgewählt ist.

4. Nukleinsäuremolekül, das ein Polypeptid nach einem der Ansprüche 1 bis 3 codiert.

5. Vektor, umfassend ein Nukleinsäuremolekül nach Anspruch 4.

6. Rekombinante Wirtszelle, umfassend einen Vektor nach Anspruch 5.

7. Verfahren zur Herstellung eines Polypeptids nach einem der Ansprüche 1 bis 3, umfassend die Schritte:
i) Züchten einer rekombinanten Wirtszelle nach Anspruch 6 unter Bedingungen, die für die Herstellung des Polypeptids geeignet sind, und
ii) Gewinnen des erhaltenen Polypeptids.

8. Verfahren nach Anspruch 7, das zudem einen Schritt umfasst, bei dem das Polypeptid gereinigt wird.

9. Verwendung eines Polypeptids nach einem der Ansprüche 1 bis 3 bei der Zellstoff-Delignifizierung.

10. Verwendung eines Polypeptids nach einem der Ansprüche 1 bis 3 bei der Textilfarbstoffbleiche.

11. Verwendung eines Polypeptids nach einem der Ansprüche 1 bis 3 bei der Abwasserentgiftung.

12. Verwendung eines Polypeptids nach einem der Ansprüche 1 bis 3 bei der xenobiotischen Entgiftung.

13. Verfahren zum Steigern der Laccase-Aktivität eines Polypeptids, umfassend eine Aminosäuresequenz, die mindestens 50% Identität zu einer Aminosäuresequenz aufweist, die aus der aus SEQ ID NO: 3, SEQ ID NO: 4 und SEQ ID NO: 5 bestehenden Gruppe ausgewählt ist, wobei die Aminosäure, die der Position 386 der Aminosäuresequenz gemäß SEQ ID NO: 3 entspricht, durch einen Glutaminrest ersetzt ist.

14. Verfahren nach Anspruch 13, wobei die drei Aminosäuren, die den Positionen 487-489 der Aminosäuresequenz gemäß SEQ ID NO: 3 entsprechen, durch eine Prolin-Tryptophan-Phenylalanin-(PWF)-Sequenz ersetzt sind.

## Revendications

1. Polypeptide ayant une activité de laccase comprenant une séquence d'acides aminés qui présente une identité, d'au moins 50%, avec une séquence d'acides aminés choisie dans le groupe constitué par les SEQ ID n° : 3, SEQ ID n° : 4 et SEQ ID n° : 5, dans laquelle un résidu de glutamine est à une position qui correspond à la position 386 de la séquence d'acides aminés selon la SEQ ID n° : 3.

2. Polypeptide, selon la revendication 1, comprenant en plus une séquence de Proline-Tryptophane-Phénylalanine (PWF) à une position qui correspond à la position 487-489 de la séquence d'acides aminés selon la SEQ ID n° : 3.

3. Polypeptide, selon la revendication 1 ou 2, comprenant une séquence d'acides aminés choisie dans le groupe constitué par la SEQ ID n° : 1 et la SEQ ID n° : 2, comprenant au moins un variant d'acide aminé choisi dans le groupe constitué par la glutamine (Q) à une position qui correspond à la position 386 de la séquence d'acides aminés selon la SEQ ID n° : 3, et une séquence de Proline-Tryptophane-Phénylalanine (PWF) à une position qui correspond à la position 487-489 de la séquence d'acides aminés selon la SEQ ID n° : 3.

4. Molécule d'acides nucléiques codant pour un polypeptide selon l'une quelconque des revendications 1 à 3.

5. Vecteur comprenant une molécule d'acides nucléiques selon la revendication 4.

6. Cellule hôte recombinante comprenant un vecteur selon la revendication 5.

7. Procédé de production d'un polypeptide, selon l'une quelconque des revendications 1 à 3, comprenant les étapes consistant à :
i. cultiver une cellule hôte recombinante, selon la revendication 6, dans des conditions appropriées pour la production du polypeptide, et
ii. récupérer le polypeptide obtenu.

8. Procédé, selon la revendication 7, comprenant en outre une étape de purification dudit polypeptide.

9. Utilisation d'un polypeptide, selon l'une quelconque des revendications 1 à 3, dans une délignification de pâte à papier.

10. Utilisation d'un polypeptide, selon l'une quelconque des revendications 1 à 3, dans un blanchiment de teintures textiles.

11. Utilisation d'un polypeptide, selon l'une quelconque des revendications 1 à 3, dans une détoxification d'eaux usées.

12. Utilisation d'un polypeptide, selon l'une quelconque des revendications 1 à 3, dans une détoxification xénobiotique.

13. Procédé d'augmentation de l'activité de laccase d'un polypeptide comprenant une séquence d'acides aminés qui présente une identité, d'au moins 50%, avec une séquence d'acides aminés choisie dans le groupe constitué par les SEQ ID n° : 3, SEQ ID n° : 4 et SEQ ID n° : 5, dans laquelle l'acide aminé, qui correspond à la position 386 de la séquence d'acides aminés selon la SEQ ID n° : 3, est remplacé par un résidu de glutamine.

14. Procédé selon la revendication 13, dans lequel les trois acides aminés, qui correspondent aux positions 487-489 de la séquence d'acides aminés selon la SEQ ID n° : 3, sont remplacés par une séquence de Proline-Tryptophane-Phénylalanine (PWF).
